# EUROPÄISCHE PATENTANMELDUNG

(11) **EP 4 675 267 A1**
(43) Veröffentlichungstag der Anmeldung: **07.01.2026**
(21) Anmeldenummer: 25177567.2
(22) Anmeldetag: 20.05.2025
(51) Int. Cl.: G01N 27/07, A47L 15/42, D06F 34/22, G01N 21/3577, G01N 21/53, D06F 103/20

(54) **SENSORVORRICHTUNG**

(30) Priorität: 01.07.2024 DE 102024118525
(71) Anmelder: emz-Hanauer GmbH & Co. KGaA, 92507 Nabburg (DE)
(72) Erfinder: Flierl, Thomas, 92507 Nabburg (DE); Brabec, Martin, 92507 Nabburg (DE)
(74) Vertreter: Hannke, Christian

(57) **Zusammenfassung**

Sensorvorrichtung insbesondere für ein wasserführendes Haushaltsgerät zur Detektion zumindest eines Prozessparameters, umfassend ein Gehäuse, in dem zumindest eine Leiterplatte, eine erste Sensoranordnung und eine zweite Sensoranordnung angeordnet sind, wobei die erste Sensoranordnung auf der zumindest einen Leiterplatte angeordnet ist, wobei die zweite Sensoranordnung entlang einer Höhenachse von der ersten Sensoranordnung beabstandet ist, wobei ein Halterelement auf der zumindest einen Leiterplatte angeordnet ist, an oder in welchem zumindest ein stiftartiges Kontaktelement angeordnet ist, wobei das zumindest eine stiftartige Kontaktelement dazu bestimmt und geeignet ist eine elektrische Verbindung zwischen der zweiten Sensoranordnung und der zumindest einen Leiterplatte bereitzustellen.

## Beschreibung

Die Erfindung betrifft eine Sensorvorrichtung insbesondere für ein wasserführendes Haushaltsgerät zur Detektion zumindest eines Prozessparameters, umfassend ein Gehäuse, in dem zumindest eine Leiterplatte, eine erste Sensoranordnung und eine zweite Sensoranordnung angeordnet sind.

Ein entsprechendes wasserführendes Haushaltsgerät ist beispielsweise eine Waschmaschine. Das wasserführende Haushaltsgerät umfasst einen Prozessraum, in welchem die zu waschenden Gegenstände aufbewahrt sind. Diesem Prozessraum wird eine Prozessflüssigkeit zugeführt, welche in der Regel Wasser ist. Der Prozessflüssigkeit können weiterhin Zusätze beigemengt sein. Derartige Zusätze können beispielsweise Waschmittel, Weichspüler oder Ähnliches sein. Bei einem Prozess, beispielsweise einem Waschvorgang, ist es nun wünschenswert bestimmte Prozessparameter zu überwachen. Ein solcher Prozessparameter ist beispielsweise die Trübheit der Prozessflüssigkeit oder das Vorhandensein von Zusätzen in der Prozessflüssigkeit. Durch die Überwachung von Prozessparametern können bestimmte Vorgänge des Prozesses, beispielsweise ein Spülvorgang automatisiert effektiver durchgeführt werden. Entsprechende Sensorvorrichtungen umfassen verschiedene Sensoren. Dadurch stellt sich die Herausforderung die entsprechenden Sensoren derart in dem Gehäuse zu platzieren, dass eine störungsfreie Detektion dieser gewährleistet wird.

Aufgabe der vorliegenden Erfindung ist es, eine Sensorvorrichtung insbesondere für ein wasserführendes Haushaltsgerät bereitzustellen, welche die oben genannten Nachteile überwindet. Ferner ist es Aufgabe dieser Erfindung ein wasserführendes Haushaltsgerät mit einer solchen Sensorvorrichtung bereitzustellen.

Die Aufgabe wird gelöst durch den Gegenstand des Anspruchs 1. Die Unteransprüche umfassen bevorzugte Ausführungsformen.

Erfindungsgemäß wird eine Sensorvorrichtung insbesondere für ein wasserführendes Haushaltsgerät zur Detektion zumindest eines Prozessparameters wasserführendes Haushaltsgerät bereitgestellt, umfassend ein Gehäuse, in dem zumindest eine Leiterplatte, eine erste Sensoranordnung und eine zweite Sensoranordnung angeordnet sind, wobei die erste Sensoranordnung auf der zumindest einen Leiterplatte angeordnet ist, wobei die zweite Sensoranordnung entlang einer Höhenachse Z von der ersten Sensoranordnung beabstandet ist, wobei ein Halterelement auf der zumindest einen Leiterplatte angeordnet ist, an oder in welchem zumindest ein stiftartiges Kontaktelement angeordnet ist, wobei das zumindest eine stiftartige Kontaktelement dazu bestimmt und geeignet ist eine elektrische Verbindung zwischen der zweiten Sensoranordnung und der zumindest einen Leiterplatte bereitzustellen.

Vorzugsweise ist das wasserführende Haushaltsgerät eine Waschmaschine oder eine Geschirrspülmaschine. Das wasserführende Haushaltsgerät umfasst vorteilhafterweise einen Prozessraum, in welchem die zu waschenden Gegenstände aufbewahrt sind. Diesem Prozessraum wird eine Prozessflüssigkeit zugeführt, welche in der Regel Wasser ist. Der Prozessflüssigkeit können vorteilhafterweise weiterhin Zusätze beigemengt sein. Derartige Zusätze können beispielsweise Waschmittel, Weichspüler oder Ähnliches sein. Ein Prozessparameter kann beispielsweise die Trübheit der Prozessflüssigkeit sein. Eine Trübheit der Prozessflüssigkeit wird beispielsweise durch Ausspülen von Schmutzpartikeln aus den oder von den zu waschenden Gegenständen verursacht. Durch eine Überwachung eines Trübheitsgrads kann ein Spülvorgang automatisiert in Abhängigkeit von der Trübheit der Prozessflüssigkeit, beziehungsweise des Verschmutzungsgrads der Prozessflüssigkeit, durchgeführt werden. Ein Prozessparameter kann auch das Vorhandensein von Zusätzen in der Prozessflüssigkeit sein. Oftmals ist es erwünscht, dass die zu waschenden Gegenstände keine Rückstände von Zusätzen beispielsweise Waschmittel nach dem Prozess aufweisen. Eine Detektion derartiger Zusätze in der Prozessflüssigkeit kann somit in einen automatisierten Spülvorgang eingebunden werden. Denkbar wäre auch, dass ein Prozessparameter eine Farbänderung der Prozessflüssigkeit ist. Unter einer Farbänderung ist im Sinne der Erfindung eine Abweichung der Farbe der Prozessflüssigkeit zu verstehen.

Die Sensorvorrichtung erstreckt sich entlang einer Höhenachse (Z), einer Längsachse (X), und einer Breitenachse (Y). Durch die Anordnung der ersten Sensoranordnung entlang der Höhenachse (Z) über der zweiten Sensoranordnung wird eine besonders effiziente Sensoranordnung bereitgestellt. Sowohl die erste Sensoranordnung auch die zweite Sensoranordnung können somit eine entsprechungssprechende Detektion durchführen, ohne die jeweils andere Sensoranordnung zu beeinflussen. Durch das Vorsehen des stiftartige Kontaktelements kann auch der zweite Sensoranordnung die benötigte elektrische Verbindung mit der Leiterplatte bereitgestellt werden.

Nach einer bevorzugten Ausführungsform umfasst die erste Sensoranordnung zumindest eine erste Sensoreinrichtung und zumindest eine zweite Sensoreinrichtung. Die zumindest eine erste Sensoreinrichtung und zumindest eine zweite Sensoreinrichtung sind entlang einer Breitenachse (Y) beabstandet. Dabei ist zwischen der zumindest einen ersten Sensoreinrichtung und zumindest einen zweiten Sensoreinrichtung ein Messraum definiert. Ein solcher Messraum ist vorzugsweise ein Volumen, welches von der Prozessflüssigkeit ausgefüllt wird oder von der Prozessflüssigkeit durchspült wird. In diesem Messraum wird somit vorteilhafterweise eine Messung des gewünschten Prozessparameters an einem Teil der Prozessflüssigkeit durchgeführt. Vorzugsweise sind die erste Sensoreinrichtung und die zweite Sensoreinrichtung auf der Leiterplatte angeordnet.

Nach einer weiteren bevorzugten Ausführungsform ist die zumindest eine erste Sensoreinrichtung eine Strahlungsquelleneinrichtung. Vorteilhafterweise emittiert die Strahlungsquelleneinrichtung zumindest eine elektromagnetische Strahlung in den Messraum. Die Wellenlange der Strahlung kann im sichtbaren Bereich bei 390 nm bis 790 nm und oder im bei 780 nm und 3 µm also im Infrarot- oder Nahinfrarotbereich liegen. Denkbar wäre auch dass die Strahlungsquelleneinrichtungen mehrere, vorzugsweise mehrere verschiedene elektromagnetische Strahlungen emittiert. Hierzu kann die Strahlungsquelleneinrichtungen mehrere Strahlungsquellen umfassen. Die Strahlungsquelleneinrichtung emittiert Strahlung vorzugsweise mit einem Welllängenbereich, welcher einer Gauß- oder Lorentzverteilung um eine Peak-Wellenlänge entspricht. Bevorzugt ist die zumindest eine zweite Sensoreinrichtung eine Detektionseinrichtung.

Nach einer weiteren bevorzugten Ausführungsform kann die Strahlungsquelleneinrichtung Strahlungsquellen umfassen, welche aus folgender Gruppe ausgewählt sein können: eine LED, ein Laser, eine Superlumineszenz-LED, ein thermischer Strahler. Denkbar wäre auch dass die Strahlungsquelleneinrichtung unterschiedliche Strahlungsquellen umfasst und eine Kombination aus der zuvor genannten Aufzählung bilden.

Nach einer weiteren bevorzugten Ausführungsform ist die zumindest eine zweite Sensoreinrichtung eine Detektionseinrichtung. Vorzugsweise detektiert die Detektionseinrichtung eine aus dem Messraum austretende elektromagnetische Strahlung. Diese aus dem Messraum austretende elektromagnetische Strahlung ist die transmittierte oder reflektierte elektromagnetische Strahlung der Strahlungsquelleneinrichtung. Beispielsweise kann die Trübheit der Prozessflüssigkeit anhand des Transmissionsgrades der elektromagnetischen Strahlung bestimmt werden.

Die Detektoreinrichtung kann vorzugsweise ausgewählt sein aus der Gruppe umfassend: ein Phototransistor, eine Photodiode, ein Bolometer. Vorteilhafterweise sind die Detektoreinrichtungen Breitbanddetektoren, welche einen breiten spektralen Bereich abdecken können. Denkbar wäre auch, dass die Detektoreinrichtungen in spezifischen Wellenlängenbereichen detektieren können. Denkbar wäre, dass die Detektoreinrichtungen hintereinanderliegende Sensoren oder aktive Schichten, so zum Beispiel Phototransistoren mit unterschiedlichen Bandlücken in den aktiven Zonen umfassen. Ebenso wäre denkbar, dass 1-Pixel oder Multi-Pixel-RGB-Kameras als Detektoreinrichtungen verwendet werden. Denkbar wären auch Kombinationen der genannten Bauteile. Vorteilhafterweise können die Detektoreinrichtungen weitere Elektronikschaltungen umfassen, um ein benötigtes Format des Informationssignals bereitzustellen. Derartige Elektronikschaltungen sind dann bevorzugt auf der Leiterplatte angeordnet.

Nach einer weiteren bevorzugten Ausführungsform umfasst, die zweite Sensoranordnung zumindest eine dritte Sensoreinrichtung und zumindest eine vierte Sensoreinrichtung. Vorzugsweise sind die zumindest eine dritte Sensoreinrichtung und zumindest eine vierte Sensoreinrichtung entlang der Breitenachse (Y) beabstandet. Vorteilhafterweise ist zwischen der zumindest einen ersten Sensoreinrichtung und zumindest einen zweiten Sensoreinrichtung der Messraum definiert. Die dritte Sensoreinrichtung und die vierte Sensoreinrichtung sind von der ersten Sensoreinrichtung und der zweiten Sensoreinrichtung entlang der Höhenachse (Z) beabstandet.

Nach einer weiteren vorteilhaften Ausführungsform ist die zweite Sensoranordnung dazu eingerichtet und bestimmt einen elektrischen Prozessparameter einer sich in dem Messraum Prozessflüssigkeit zu bestimmen. Vorteilhafterweise sind die zumindest eine dritte Sensoreinrichtung und die zumindest eine vierte Sensoreinrichtung als Elektroden ausgebildet. Der elektrische Prozessparameter ist bevorzugt ein elektrischer Leitwert und/oder eine elektrische Kapazität.

Es befindet sich somit die Prozessflüssigkeit in dem Messraum zwischen der als Elektrode ausgebildeten ersten Sensoreinrichtung und der als Elektrode ausgebildeten zweiten Sensoreinrichtung. Die wasserbasierte Prozessflüssigkeit ist in der Regel elektrisch leitend. Durch Anlegen einer Spannung an die Elektroden kann somit ein Leitwert beziehungsweise ein Widerstand der zwischen den Elektroden befindlichen Prozessflüssigkeit bestimmt werden. Dieser Leitwert ist abhängig von eventuellen sich in der Prozessflüssigkeit befindlichen Zusätzen. Bei Kenntnis des Leitwerts der Prozessflüssigkeit ohne Zusatz (also Wasser) kann bestimmt werden, ob die Prozessflüssigkeit entsprechende Zusätze aufweist. Ist dies der Fall kann beispielsweise ein weiterer Spülvorgang initiiert werden.

Nach einer weiteren bevorzugten Ausführungsform sind die dritte Sensoreinrichtung und die vierte Sensoreinrichtung dazu bestimmt und eingerichtet sind die Prozessflüssigkeit in dem Messraum direkt zu kontaktieren. Vorzugsweise sind die dritte Sensoreinrichtung und die vierte Sensoreinrichtung im Wesentlichen stabartig ausgebildet.

Durch die erfindungsgemäße Anordnung der zweiten Sensoranordnung entlang der Höhenachse (Z) über der ersten Sensoranordnung kann somit sowohl zumindest ein elektrischer Prozessparameter als auch ein Prozessparameter, welcher vorzugsweise mit optischen Sensoreinrichtungen bestimmbar ist, bestimmt werden. Durch diese Anordnung können vorteilhafterweise die erste Sensoreinrichtung und die zweite Sensoreinrichtung einander gegenüberliegend angeordnet werden. Vorteilhafterweise kann somit auf Lichtleiter und/oder weitere optische Komponenten verzichtet werden beziehungsweise es kann die Anzahl solcher optischer Komponenten erheblich reduziert werden. Hierdurch wird eine sehr einfache Anordnung bereitgestellt, welche kostengünstiger und kompakter produziert werden kann, da optische Komponenten und gegebenenfalls Lichtleiter nicht oder den geringeren Maßen benötigt werden.

Nach einer weiteren bevorzugten Ausführungsform ist das zumindest eine stiftartige Kontaktelement an der Leiterplatte angeordnet. Insbesondere ist das stiftartige Kontaktelement an der Leiterplatte befestigt. Dies kann beispielsweise mittels einer Lötverbindung erfolgen. Vorteilhafterweise erstreckt sich das stiftartige Kontaktelement ausgehend von der Leiterplatte entlang der Höhenachse (Z). Bevorzugt ist der zumindest einen dritten Sensoreinrichtung und der zumindest einen vierten Sensoreinrichtung jeweils zumindest ein stiftartiges Kontaktelement zugeordnet. Demnach entspricht die Anzahl der Sensoreinrichtungen der zweiten Sensoranordnung der Anzahl der stiftartigen Kontaktelemente. Vorzugsweise sind somit zwei stiftartige Kontaktelemente vorgesehen. Vorzugsweise weisen ein erstes stiftartiges Kontaktelement eine elektrisch leitende Verbindung mit der dritten Sensoreinrichtung und ein zweites stiftartiges Kontaktelement eine elektrisch leitende Verbindung mit der vierten Sensoreinrichtung auf.

Nach einer weiteren bevorzugten Ausführungsform ist jeweils zwischen einem stiftartigen Kontaktelement und einer Sensoreinrichtung der zweiten Sensoranordnung ein zweites Kontaktelement angeordnet. Das zweite Kontaktelement stellt eine elektrische Verbindung zwischen dem jeweiligen stiftartigen Kontaktelement und der jeweiligen Sensoreinrichtung bereit. Vorzugsweise ist das zweite Kontaktelement als ein elastisches Element ausgebildet. Vorzugsweise besteht das elastische Element aus einem elektrisch leitenden Material. Bevorzugt ist das elastische Element ein Federelement. Das Federelement kann eine Spiralfeder eine Torsionsfeder oder ähnliches sein.

Vorzugsweise ist das elastische Element mit einer Vorspannung zwischen dem stiftartigen Kontaktelement und der jeweiligen Sensoreinrichtung der zweiten Sensoranordnung angeordnet. Durch diese Vorspannung wird ein entsprechender Kontakt und somit die elektrische Verbindung zwischen dem stiftartigen Kontaktelement und der jeweiligen Sensoreinrichtung gewährleistet. Weiterhin hat das Vorsehen eines elastischen Elements den Vorteil, dass Größenänderungen der Sensoreinrichtung, beispielsweise eine Ausdehnung oder eine Verkleinerung durch eine Änderung der Vorspannung ausgeglichen werden. Derartige Größenänderungen können beispielsweise durch thermische Einflüsse aufgrund des direkten Kontakts der Sensoreinrichtung mit der Prozessflüssigkeit auftreten. Vorzugsweise ist sind die Sensoreinrichtungen der zweiten Sensoranordnung aus einem Metall gefertigt, welches einen entsprechenden thermischen Ausdehnungskoeffizienten aufweist. Konventionelle elektrische Verbindungen können durch derartige thermische Ausdehnungen mit der Zeit zerstört werden. Durch das Vorsehen eines elektrisch leitenden elastischen Elements ist somit eine langlebige elektrische Verbindung gewährleistet.

Nach einer weiteren vorteilhaften Ausführungsform weist das Gehäuse einen oberen Abschnitt auf, in welchem zwei turmartige Elemente ausgebildet sind. Vorzugsweise sind die turmartigen Elemente entlang der Breitenachse (Y) sich gegenüberliegend angeordnet. Vorteilhafterweise sind die beiden turmartigen Elemente und der obere Abschnitt einstückig oder einteilig ausgebildet. Vorzugsweise sind die zumindest eine erste Sensoreinrichtung und zumindest eine zweite Sensoreinrichtung jeweils in einem der turmartigen Elemente angeordnet. Vorzugsweise weist die Leiterplatte zwei fingerartige Abschnitte auf, welche sich entlang einer Längsrichtung (X) erstrecken. Vorzugsweise ist jeweils ein fingerartiger Abschnitt in einem turmartigen Element angeordnet. Zwischen den beiden turmartigen Elementen befindet sich somit vorteilhafterweise der Messraum, in welchem sich wiederum die Prozessflüssigkeit befindet oder durch welchen die Prozessflüssigkeit strömt.

Vorzugsweise sind die zumindest eine erste Sensoreinrichtung auf einem der fingerartigen Abschnitte der Leiterplatte und die zumindest eine zweite Sensoreinrichtung auf dem weiteren fingerartigen Abschnitt der Leiterplatte angeordnet. Die beiden Sensoreinrichtungen sind somit sich gegenüberliegend angeordnet.

Vorzugsweise ist der obere Abschnitt des Gehäuses zumindest aber die turmartigen Abschnitte aus einem Material gefertigt, welches für die elektromagnetische Strahlung der zumindest einen Strahlungsquelleneinrichtung im Wesentlichen transparent ist. Durch den Ausdruck "im Wesentlichen transparent" soll ausgedrückt werden, dass ein Durchgang durch das Gehäuse für die entsprechenden Strahlungen keine nennenswerten Absorptions- und/oder Reflexionsverluste verursacht.

Weiter bevorzugt ist der obere Abschnitt des Gehäuses derart ausgebildet, dass dieses gegen-über der Flüssigkeit abgedichtet ist. Vorzugsweise ist das Gehäuse aus einem oder mehreren Kunststoffen hergestellt. Vorzugsweise ist das Gehäuse mittels eines Kunststoffspritzverfahren hergestellt

Nach einer weiteren vorteilhaften Ausführungsform weisen die turmartigen Elemente jeweils eine Aufnahme zur Anordnung einer Sensoreinrichtung der zweiten Sensoranordnung auf. Vorzugsweise sind die dritte Sensoreinrichtung und die vierte Sensoreinrichtung jeweils in einer Aufnahme eines turmartigen Elements angeordnet. Vorzugsweise erstrecken sich die dritte Sensoreinrichtung und die vierte Sensoreinrichtung jeweils durch eine Öffnung in dem oberen Abschnitt des Gehäuses. Dabei ist es von Vorteil, dass die dritte Sensoreinrichtung und die vierte Sensoreinrichtung jeweils ein Dichtelement aufweisen, welches zwischen der jeweiligen Sensoreinrichtung und der jeweiligen Öffnung angeordnet ist. Somit sind die dritte Sensoreinrichtung sowie die vierte. Sensoreinrichtung in direkten Kontakt mit der Prozessflüssigkeit. Durch die Abdichtung mit dem jeweiligen Dichtelement ist das Innere des Gehäuses geschützt.

Nach einer weiteren vorteilhaften Ausführungsform schließt ein unterer Abschnitt des Gehäuses an den oberen Abschnitt des Gehäuses an. Dabei ist bevorzugt in einem oberen Bereich des unteren Abschnitts des Gehäuses ein Dichtelement an einer äußeren Wandung des unteren Abschnitts angeordnet. Vorteilhafterweise das Dichtelement vollständig um den Umfang des unteren Abschnitts umflaufend ausgebildet. Die Sensorvorrichtung ragt vorteilhafterweise in den Prozessraum des Haushaltsgeräts hinein. Dabei ist der obere Abschnitt vorzugweise in dem Prozessraum angeordnet. Der untere Abschnitt ist vorzugsweise dazu bestimmt und geeignet, eine signaltechnische Verbindung mit dem Haushaltsgerät, insbesondere mit einer Steuerungseinrichtung des Haushaltsgeräts zu ermöglichen. Vorteilhafterweise weist der untere Abschnitt eine Öffnung auf, durch welche ein Steckerelement in das Gehäuse einführbar ist. Durch das Dichtelement ist die Anordnung der Sensoreinrichtung in dem Prozessraum abgedichtet.

Vorzugsweise ist eine Steuerungseinrichtung vorgesehen, welche signaltechnisch mit der ersten Sensoranordnung und der zweiten Sensoranordnung verbunden ist. Vorteilhafterweise ist die Steuerungseinrichtung dem Haushaltsgerät zugehörig. Denkbar wäre jedoch auch, dass eine Steuerungseinrichtung dem der Sensorvorrichtung zugehörig ist. Eine solche der Sensorvorrichtung zugehörige Steuerungseinrichtung könnte dann mit einer Steuerungseinrichtung des Haushaltsgeräts signaltechnisch verbunden sein.

Nach einer weiteren vorteilhaften Ausführungsform weist das Halterelement einen Rastabschnitt auf, welcher ein einem an der Leiterplatte anordenbaren Steckerelement einrastbar ist. Ein solches Steckerelement wird vorteilhafterweise durch die Öffnung des unteren Abschnitts des Gehäuses eingeführt. Vorzugsweise weist die Leiterplatte Kontaktflächen, auf welche mit dem Steckerelement in Kontakt treten. Bevorzugt wird das Steckerelement auf die Leiterplatte aufgeschoben und umgreift somit vorteilhafterweise einen Teil der Leiterplatte, welcher die entsprechenden Kontaktflächen umfasst. Durch den Rastabschnitt wird eine lösbare formschlüssige Verbindung mit dem Steckerelement bereitgestellt. Das Steckerelement hat somit einen sicheren Halt an der Leiterplatte.

Nach einer weiteren vorteilhaften Ausführungsform weist das Halterelement zumindest einen Armabschnitt aufweist. Vorzugsweise weist der Armabschnitt ein Befestigungselement auf. Bevorzugt befestigt das Befestigungselement den Armabschnitt an der Leiterplatte. Hierzu kann beispielsweise eine formschlüssige Verbindung zwischen dem Befestigungselements und der Leiterplatte vorgesehen sein denkbar wären auch anderweitige gegebenenfalls lösbare Verbindungen beispielsweise Schnappverbindungen. Vorzugsweise ist das Befestigungselements hakenartig ausgebildet und greift durch eine Bohrung in der Leiterplatte hindurch. Denkbar wäre auch das das Befestigungselement als eine Ausnehmung oder eine Bohrung in dem Armelement ausgebildet ist, welches ein komplementäres Befestigungselement von der Leiterplatte aufnimmt. Durch den Armabschnitt mit dem Befestigungselements wird eine entsprechende mechanische Festigkeit gewährleistet. Eventuell auftretende Kräfte bei einem Ein- bzw. Ausrasten des Rastabschnitts des Halteelements an dem Steckerelement das Halterelement nicht beeinflusst wird.

Die Aufgabe wird weiterhin gelöst durch ein Haushaltsgerät umfassend zumindest eine Sensorvorrichtung nach einem der vorhergehend beschriebenen Ausführungsformen. Das Haushaltsgerät kann dabei mit allen bereits obig im Rahmen der Sensorvorrichtung beschriebenen Merkmalen einzeln oder in Kombination miteinander ausgestattet sein und umgekehrt.

Vorteilhafterweise ist das Haushaltsgerät ein wasserführende Haushaltsgerät beispielsweise eine Waschmaschine oder eine Geschirrspülmaschine.

Weitere Vorteile, Ziele und Eigenschaften der vorliegenden Erfindung werden anhand nachfolgender Beschreibungen der anliegenden Figuren erläutert. Gleichartige Komponenten können in den verschiedenen Ausführungsformen gleiche Bezugszeichen aufweisen.

In den Figuren zeigen:
- Fig.1: ein Haushaltsgerät mit einer Sensorvorrichtung;
- Fig. 2: eine Sensorvorrichtung nach einer Ausführungsform;
- Fig. 3: eine Schnittansicht einer Sensorvorrichtung nach einer Ausführungsform;
- Fig. 4: einen Teil einer Sensorvorrichtung nach einer Ausführungsform;
- Fig. 5: einen Teil einer Sensorvorrichtung nach einer Ausführungsform;
- Fig. 6: ein Haltelement nach einer Ausführungsform;
- Fig. 7: Sensoreinrichtungen nach einer Ausführungsform.

In den Figuren sind gleiche Bauteile jeweils mit den entsprechenden Bezugszeichen zu verstehen. Zur besseren Übersichtlichkeit können manchen Figuren Bauteile nicht mit einem Bezugszeichen versehen sein, jedoch an anderer Stelle bezeichnet worden sind.

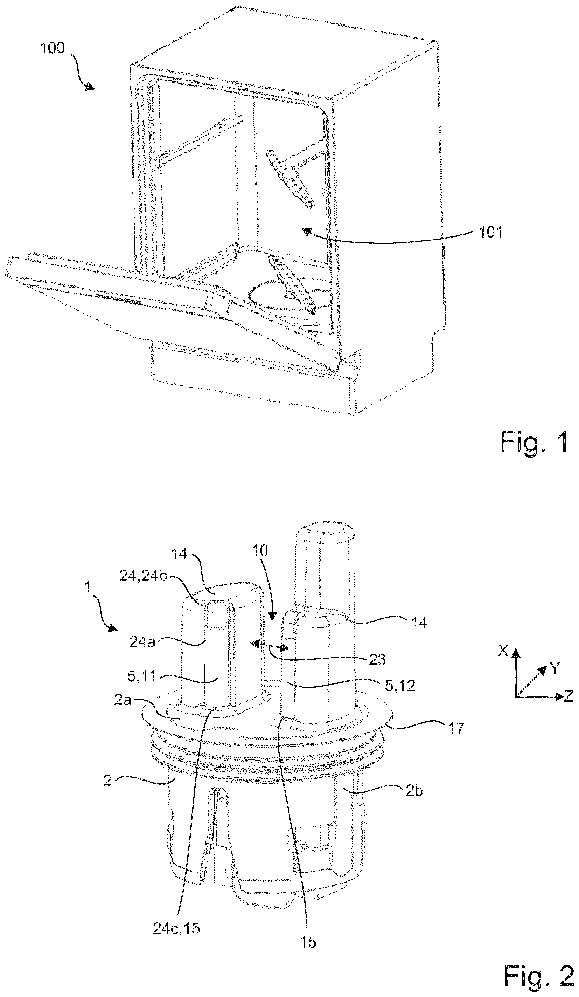

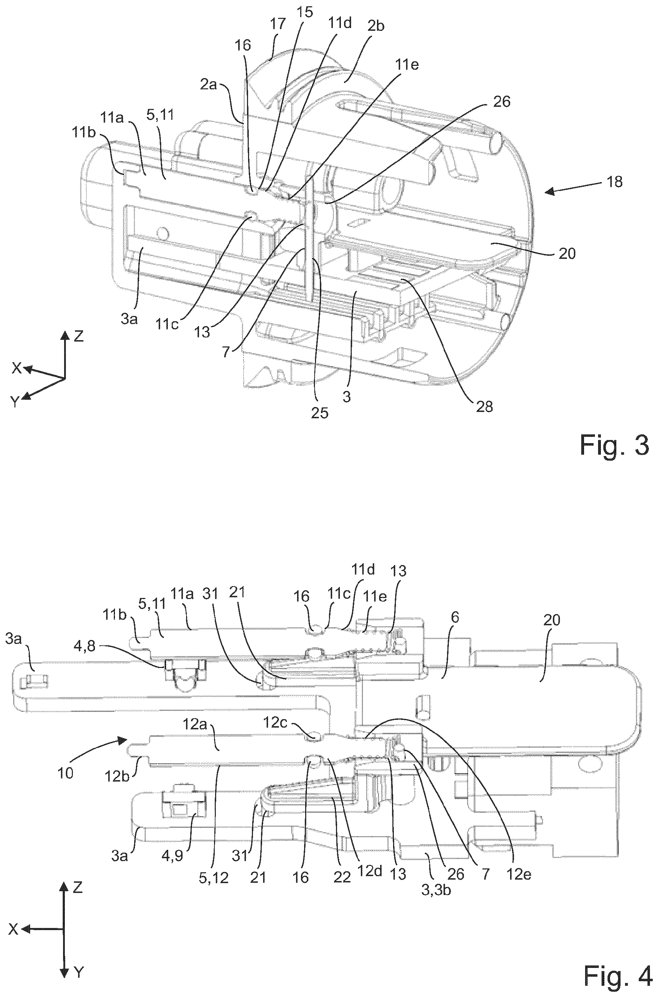

In den Figuren 2 bis 6 ist eine Sensorvorrichtung 1 insbesondere für ein wasserführendes Haushaltsgerät 100 zur Detektion zumindest eines Prozessparameters gezeigt. Die Sensorvorrichtung 1 umfasst ein Gehäuse 2, in dem zumindest eine Leiterplatte 3, eine erste Sensoranordnung 4 und eine zweite Sensoranordnung 5 angeordnet sind. Die erste Sensoranordnung 4 ist auf der zumindest einen Leiterplatte 3 angeordnet, wobei die zweite Sensoranordnung 5 entlang einer Höhenachse Z von der ersten Sensoranordnung 4 beabstandet ist, wobei ein Halterelement 6 auf der zumindest einen Leiterplatte 3 angeordnet ist, an oder in welchem zumindest ein stiftartiges Kontaktelement 7 angeordnet ist, wobei das zumindest eine stiftartige Kontaktelement 7 dazu bestimmt und eingerichtet ist eine elektrische Verbindung zwischen der zweiten Sensoranordnung 5 und der zumindest einen Leiterplatte 3 bereitzustellen.

Figur 1 zeigt wasserführendes Haushaltsgerät 100 umfassend einen Prozessraum 101, in welchem eine Prozessflüssigkeit einleitbar ist. Das Haushaltsgerät 100 kann beispielsweise als ein Geschirrspüler, als eine Waschmaschine oder dergleichen ausgebildet sein kann. Die Sensorvorrichtung 1 ist in dem Haushaltsgerät 1 angeordnet. Dabei ist es bevorzugt vorgesehen, dass die Sensorvorrichtung 1 zumindest teilweise mit der Prozessflüssigkeit 3 in Kontakt ist.

In Figur 2 ist Sensorvorrichtung 1 dargestellt, welche ein beispielhaftes Gehäuse 2 umfasst. Das Gehäuse 2 umfasst zwei turmartige Elemente 14, welche mit einem Abstand 23 voneinander beabstandet sind. Zwischen den turmartigen Abschnitten 14 befindet sich ein Zwischenbereich, welcher als Messraum 10 definiert ist. In dem Messraum 10 befindet sich während eines Prozesses, beispielsweise eine Waschprozesses, Prozessflüssigkeit, beziehungsweise es wird während des Prozesses die Prozessflüssigkeit durch den Messraum 10 gespült.

Das Gehäuse 2 umfasst einen oberen Abschnitt 2a und einen unteren Abschnitt 2b. Der obere Abschnitt 2a geht integral in den unteren Abschnitt über. Insbesondere ist das Gehäuse 2, als der obere Abschnitt 2a und der untere Abschnitt 2b einstückig oder einteilig ausgebildet. Die turmartigen Elemente 14 sind einteilig oder einstückig mit dem oberen Abschnitt 2a ausgebildet.Unter einer einteiligen Ausgestaltung ist hierbei zu verstehen, dass sämtliche Abschnitte aus einem einzigen und einheitlichen Teil gefertigt werden. Unter einer einstückigen Ausgestaltung ist hierbei zu verstehen, dass sämtliche Abschnitte zwar nicht aus einem einzigen und einheitlichen Teil gefertigt werden, jedoch nicht nur fest, sondern so innig miteinander verbunden sind, dass sie nicht als mehrere aneinander gefügte Bauteile erscheinen und jedenfalls nicht mehr voneinander gelöst werden können, ohne dass dabei diese dabei zerstört werden. Das Gehäuse 2 ist dabei aus zumindest einem Kunststoff hergestellt.

Vorteilhafterweise ragt das Gehäuse 2 teilweise in den in dem Prozessraum 101. Hierzu ist eine Öffnung in einer Wandlung des Prozessraums 101 vorgesehen. Der untere Abschnitt 2b ist im Wesentlichen als kreisförmige Hohlzylinder ausgebildet. In einem oberen Bereich des unteren Abschnitts 2b ist ein Dichtelement 17 angeordnet. Dieses Dichtelement 17 weist zumindest eine Dichtlippe auf. Dieses Dichtelement 17 sorgt dabei für einen dichten Abschluss der Öffnung in dem Prozessraum 101. Somit befindet sich der obere Abschnitt 2a mit den beiden turmartigen Elementen 14 innerhalb des Prozessraums 101 und der untere Abschnitt 2b ist außerhalb des Prozessraums 101 angeordnet.

Zur Detektion der Prozessparameter sind die erste Sensoranordnung 4 und eine zweite Sensoranordnung 5 vorgesehen. Denkbar wäre natürlich auch, dass weitere Sensoranordnungen vorgesehen sind.

Die erste Sensoranordnung 4 ist eine optische Sensoranordnung und umfasst zumindest eine erste Sensoreinrichtung 8 in Form einer Strahlungsquelleneinrichtung und zumindest eine zweite Sensoreinrichtung 9 in Form einer Detektionseinrichtung. Die zumindest eine erste Sensoreinrichtung 8 in Form einer Strahlungsquelleneinrichtungen emittiert zumindest eine elektromagnetische Strahlung in den Messraum 10. Denkbar wäre auch das mehrere elektromagnetische Strahlungen mit unterschiedlichen Wellenlängenbereichen in den Messraum 10 emittiert werden. Die elektromagnetischen Strahlungen können Wellenlängen im sichtbaren Bereich bei 390 nm bis 790 nm und oder bei 780 nm und 3 µm also im Infrarot- oder Nahinfrarotbereich aufweisen. Die zumindest eine elektromagnetische Strahlung der Strahlungsquelleneinrichtungen wird durchtritt die Prozessflüssigkeit im Messraum 10 (Transmissionsgeometrie) und/oder wird im Messraum reflektiert (Reflexionsgeometrie). Die aus dem Messraum 10 ausgetretene elektromagnetische Strahlung wird dann von der zumindest eine zweite Sensoreinrichtung 9 in Form einer Detektionseinrichtung detektiert. Insbesondere bei der Transmissionsmessung ist es von Vorteil, dass die zumindest eine erste Sensoreinrichtung 8 in Form einer Strahlungsquelleneinrichtung und die zumindest eine zweite Sensoreinrichtung 9 in Form einer Detektionseinrichtung entlang der Breitenachse Y gegenüberliegend angeordnet sind.

Die zumindest eine erste Sensoreinrichtung 8 und die zumindest eine zweite Sensoreinrichtung 9 sind jeweils in einem der turmartigen Elemente 14 angeordnet. Weiterhin sind die zumindest eine erste Sensoreinrichtung 8 und die zumindest eine zweite Sensoreinrichtung 9 auf der Leiterplatte 3 angeordnet. Hierzu umfasst die Leiterplatte 2 fingerartige Abschnitte 3a, welche sich entlang der Längsachse X erstrecken. Jeder der fingerartigen Abschnitte 3a erstreckt sich jeweils in einen der turmartigen Elemente 14. Weiterhin münden die fingerartigen Abschnitte 3a münden in einem Hauptabschnitt 3b der Leiterplatte 3.

Die turmartigen Elemente 14 sind somit zum Schutz der optischen Komponenten vor einem direkten Kontakt mit der Prozessflüssigkeit angedacht. Die turmartigen Elemente 14, gegebenenfalls der gesamte obere Abschnitt 2a sind dabei aus einem Material gefertigt, welches im Wesentlichen transparent für die oben genannte elektromagnetische Strahlung ist. Der untere Abschnitt 2b des Gehäuses 2 muss nicht zwangsläufig, kann aber auch transparent für die jeweiligen Strahlungen ausgebildet sein.

Die zweite Sensoranordnung 5 umfasst zumindest eine dritte Sensoreinrichtung 11 und zumindest eine vierte Sensoreinrichtung 12. Die zumindest eine dritte Sensoreinrichtung 11 und die zumindest eine vierte Sensoreinrichtung 12 sind als Elektroden ausgebildet. Demnach ist die zweite Sensoranordnung 5 dazu eingerichtet und bestimmt, einen elektrischen Prozessparameter einer sich in dem Messraum 10 befindlichen Prozessflüssigkeit zu bestimmen. Der elektrische Prozessparameter ist dabei ein elektrischer Leitwert und/oder eine elektrische Kapazität. Derartige Leitwertmessungen dienen beispielsweise zur Feststellung, ob und zu welchem Grad die Prozessführungsflüssigkeit mit einem Zusatz versetzt ist.

Zur Detektion des Leitwerts wird eine Spannung zwischen den beiden Elektronen angelegt. Hierzu ist es notwendig, dass die dritte Sensoreinrichtung 11 und die vierte Sensoreinrichtung 12 in direkten Kontakt mit der Prozessflüssigkeit stehen.

In den Figuren die 2, 3, 4 und 5 ist erkennbar das die dritte Sensoreinrichtung 11 und die vierte Sensoreinrichtung 12 im Wesentlichen stabartig ausgebildet sind. Die dritte Sensoreinrichtung 11 und die vierte Sensoreinrichtung 12 erstrecken sich entlang der Höhenachse Z jeweils über einem der fingerartigen Abschnitte 3a der Leiterplatte 3 und befinden sich somit jeweils entlang der Höhenachse Z über einer der Sensoreinrichtungen 8, 9 der ersten Sensoranordnung 4. Nach der in den Figuren dargestellten Ausführungsform befindet sich somit die dritte Sensoreinrichtung 11 über der ersten Sensoreinrichtung 8. Die vierte Sensoreinrichtung 12 befindet sich über der zweiten Sensoreinrichtung 9. Somit sind die dritte Sensoreinrichtung 11 und die erste Sensoreinrichtung 8 entlang der Breitenachse Y von der vierten Sensoreinrichtung 12 der zweiten Sensoreinrichtung 9 beabstandet.

Die beiden Sensoreinrichtungen 11,12 der zweiten Sensoranordnung 5 weisen einen Hauptabschnitt 11a, 12a auf, welcher im Wesentlichen kreiszylinderartig ausgebildet ist. Denkbar wären aber auch noch anderweitige geometrische Formen beispielsweise quaderförmig etc.. An einem vorderen Ende der beiden Sensoreinrichtungen 11,12 schließt an den Hauptabschnitt 11a, 12a ein Befestigungsabschnitt 11b, 12b an. Der Befestigungsabschnitt 11b, 12b ist stiftartig ausgebildet und weist einen kleineren Durchmesser als der Hauptabschnitt 11a, 12a auf.

Gegenüberliegend des Befestigungsabschnitts 11b, 12b geht der Hauptabschnitt 11a, 12a über in einen Aufnahmeabschnitt 11c, 12c. Der Aufnahmeabschnitt 11c, 12c, welcher einen ein Dichtelement 16 in Form eine O-Rings aufnimmt, ist muldenartig ausgebildet und weist somit einen geringeren Durchmesser als der Hauptabschnitt 11a, 12a auf. Anschließend an den Aufnahmeabschnitt 11c, 12c ist ein Übergangsabschnitt 11d, 12d vorgesehen. Dieser Übergangsabschnitt 11d, 12d weist zunächst einen Durchmesser auf, welcher im Wesentlichen dem Durchmesser des Hauptabschnitts 11a, 12a entspricht. Im weiteren Verlauf entlang der Längsachse X in Richtung zu einem hinteren Ende der jeweiligen Sensoreinrichtung 11, 12 hin nimmt der Durchmesser des Übergangsabschnitts 11d, 12d kontinuierlich ab. Der Übergangsabschnitt 11d, 12d mündet schließlich in einem Endabschnitt 11e, 12e, welcher einen kleineren Durchmesser als der Hauptabschnitt 11a, 12a aufweist.

Die turmartigen Elemente 14 weisen jeweils eine Aufnahme 24 auf, in welcher jeweils eine Sensoreinrichtung 11, 12 der zweiten Sensoranordnung 5 aufgenommen ist. Die Aufnahme 24 weist einen ersten Abschnitt 24a, in welchem der Hauptabschnitt 11a, 12a der jeweiligen Sensoreinrichtung 11,12 angeordnet ist. Dieser erste Abschnitt 24a weist einen kreisbogenartigen Querschnitt auf, sodass ein Teil der Mantelfläche des Hauptabschnitts 11a, 12a an dem ersten Abschnitt 24a der Aufnahme 24 anliegt. Der verbleibende Teil der Mantelfläche ist somit frei liegend und kann die Prozessflüssigkeit kontaktieren. Weiterhin weist die Aufnahme 24 einen zweiten Abschnitt 24b auf, in welchem der stiftartige ausgebildete Endabschnitt 11b, 12b aufgenommen ist. Der Endabschnitt 11b, 12b ist vollends umgeben von dem zweiten Abschnitt 24b.

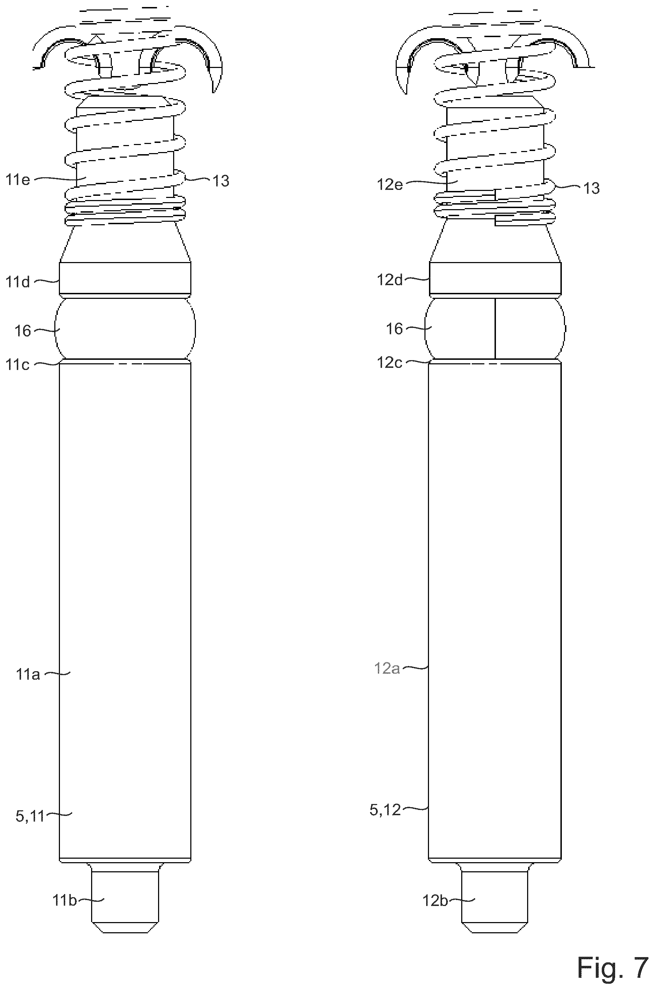

Die dritte Sensoreinrichtung 11 und die vierte Sensoreinrichtung 12 erstrecken sich jeweils durch eine Öffnung 15 in dem oberen Abschnitt 2a des Gehäuses 2.Diese Öffnung 15 ist in einem dritten Abschnitt 24c Aufnahme 24 vorgesehen. Der dritte Abschnitt 24c ist als ein holzylinderartiges Lagerelement, mit einem kreisförmigen Querschnitt ausgebildet. Das Dichtelement 16, welches als O-Ring ausgebildet ist und in dem Aufnahmeabschnitt 11c, 12c der Sensoreinrichtungen 11, 12 angeordnet ist liegt an einer inneren Wandung des dritte Abschnitts 24c an, wodurch eine Abdichtung gegenüber dem Inneren des unteren Abschnitts 2b des Gehäuses 2 gewährleistet wird. Die jeweilige Sensoreinrichtung 11, 12 wird somit durch den ersten Abschnitt 24a und den dritten Abschnitt 24c der jeweiligen Aufnahme 24 gehalten. Die dritte Sensoreinrichtung 11 und die vierte Sensoreinrichtung 12 sind in Figur 7 näher dargestellt.

Das zumindest eine stiftartige Kontaktelement 7 ist an der Leiterplatte 3 angeordnet und erstreckt sich ausgehend von der Leiterplatte 3 entlang der Höhenachse Z. erstreckt. Die elektrische Verbindung zwischen dem stiftartigen Kontaktelement 7 und der Leiterplatte 3 erfolgt beispielsweise über eine Lötverbindung. Der zumindest einen dritten Sensoreinrichtung 11 und der zumindest einen vierten Sensoreinrichtung 12 ist jeweils zumindest ein stiftartiges Kontaktelement 7 zugeordnet ist. Nach den in den Figuren dargestellten Ausführungsformen sind somit zwei stiftartige Kontaktelemente 7 vorgesehen.

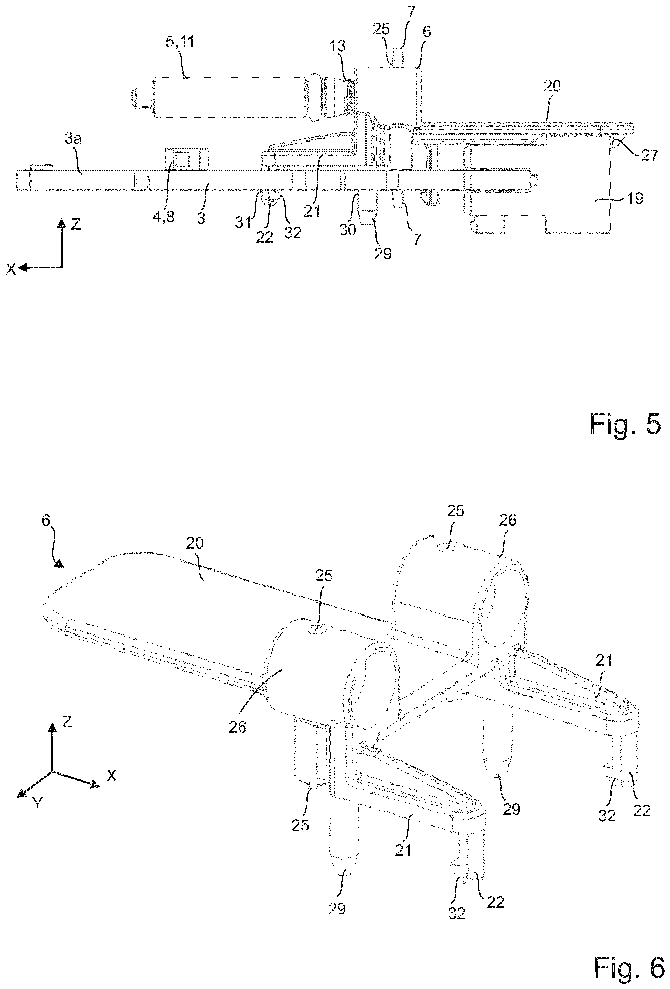

Die stiftartige Kontaktelemente 7 sind werden weiterhin durch das Halterelement 6 gehalten. Hierzu ist in dem Halterelement 6 ein erster Durchgangskanal 25 vorgesehen. Nach Figur 5 ragt das Kontaktelement 7 sowohl über das Halterelement 6, beziehungsweise aus dem ersten Durchgangskanal 25, als auch über die Leiterplatte 3 hinaus.

Jeweils zwischen einem stiftartigen Kontaktelement 7 und einer Sensoreinrichtung 11, 12 der zweiten Sensoranordnung 5 ist ein zweites Kontaktelement 13 angeordnet. Das zweite Kontaktelement 13 stellt eine elektrische Verbindung zwischen dem jeweiligen stiftartigen Kontaktelement 7 und der jeweiligen Sensoreinrichtung 11, 12 bereit. Das zweite Kontaktelement 13 ist als ein elastisches Element insbesondere ein Federelement ausgebildet. Das zweite Kontaktelement 13 ist teilweise auf dem Endabschnitt 11e, 12e der jeweiligen Sensoreinrichtung 11,12 angeordnet. Weiterhin ist das zweite Kontaktelement 13 an dem stiftartigen Kontaktelement 7 anliegend, so dass eine elektrische Verbindung zwischen dem stiftartigen Kontaktelement 7 der jeweiligen Sensoreinrichtung 11, 12 besteht.

Ferner stellt das zweite Kontaktelement 13, welches vorliegend aus einer Spiralfederelement ausgebildet ist, eine Vorspannung zwischen dem stiftartigen Kontaktelement 7 und der jeweiligen Sensoreinrichtung 11,12 bereit. Die Vorspannung ist bedingt dadurch, dass das stiftartige Kontaktelement 7 durch das Halterelement 6 befestigt ist und die jeweilige Sensoreinrichtung 11, 12 durch die Aufnahme 24 befestigt ist. Da die Sensoreinrichtungen 11,12 vorzugsweise aus einem Metall gefertigt sind, kann eine Temperaturänderung der Prozessflüssigkeit eine thermische Ausdehnung der jeweiligen Sensoreinrichtung 11,12 verursachen. Derartige thermische Ausdehnungen können durch das zweite Kontaktelement 13 ausgeglichen werden. Es ändert sich die lediglich die Vorspannung. Eine mechanisch feste leitende Verbindung würde durch derartige thermische Ausdehnungen beschädigt werden.

Das Halterelement 6 weist zwei hohlzylinderartige zweite Aufnahmen 26 auf, welche einen kreisförmigen Querschnitt aufweisen. Die zweiten Aufnahmen 26 umfassen einen zweiten Durchgangskanal 26a, welcher sich entlang der Längsachse X erstreckt. In diese zweiten Aufnahmen 26, beziehungsweise die zweiten Durchgangskanäle 26a ragt der jeweilige Endabschnitt 11b, 12b des jeweiligen Sensorelements 11,12. Der jeweilige erste Durchgangskanal 25, in welchem das stiftartige Kontaktelement angeordnet ist, verläuft senkrecht, also im Wesentlichen parallel zu der Höhenachse Z zu dem jeweiligen zweiten Durchgangskanal 26.

In Figur 6 ist das Halterelement 6 dargestellt. Neben dem bereits obig beschriebenen Komponenten weist das Halterelement 6 weiterhin einen Rastabschnitt 20 auf, welcher an einem an der Leiterplatte 3 anordenbaren Steckerelement 19 einrastbar ist. Der Rastabschnitt 20 ist plattenartig ausgebildet und verfügt an einer unteren Seite über einen Rastvorsprung 27, welcher an dem Steckerelement 19 eingreifen kann. Der Rastabschnitt 20 weist eine ausreichende Elastizität auf, so dass dieser derart deformierbar ist, um die Rastverbindung zu dem Steckerelement 19 zu lösen, beziehungsweise den Rastvorsprung entlang der Höhenachse Z ausreichend weit anzuheben.

Das Steckerelement 19 umgreift einen Abschnitt der Leiterplatte 3. Die Leiterplatte 19 verfügt über entsprechende Kontaktflächen 28, welche mit dem Steckerelement 19 in Kontakt sind. Durch das Steckerelement 19 kann eine Verbindung zu dem Haushaltsgerät 100, beziehungsweise einer Steuereinrichtung des Haushaltsgeräts 100, kabelgebunden hergestellt werden

Zur mechanischen Befestigung des Halteelements 6 an der Leiterplatte 6 umfasst das Halteelements 6 zwei stiftartige Elemente 29, welche im Wesentlichen entlang der Höhenachse Z unterhalb den zweiten Aufnahmen 26 angeordnet sind. Diese stiftartigen Elemente 29 ragen durch entsprechende erste Bohrungen 30 der Leiterplatte 3.

Weiterhin weist das Halterelement 6 zumindest einen Armabschnitt 21 auf. Vorliegend sind zwei Armabschnitte 21 vorgesehen. Der Armabschnitt weist ein Befestigungselement 22 auf, welches dazu vorgesehen ist den Armabschnitt 21 und somit das Halterelement 6 an der Leiterplatte 3 zu befestigen. Das Befestigungselement 22 weist einen stiftartigen Abschnitt auf, an dessen Ende ein hakenartiger Vorsprung 32 angeordnet ist. Die Befestigungselemente 22 ragen durch zweite Bohrungen 31. Die hakenartigen Vorsprünge 32 greifen dann an einer Unterseite der Leiterplatte 3 ein.

Denkbar wäre das Vorsehen einer Elektronikschaltung, welche das elektrische Signal der Detektionseinrichtung in die von der Steuerungseinrichtung benötigten Informationssignale umwandelt. Entsprechende Informationssignale können ein elektrischer Strom, eine elektrische Spannung, ein P WM (Pulsweiten Moduliertes) - Signal oder ein AM (Amplituden Moduliertes) - Signal sein. Diese Elektronikschaltung ist vorzugsweise auf der Leiterplatte angeordnet. Die Leiterplatte 3 ist beispielsweise eine PCB (Printed Circuit Board).

Die Steuerungseinrichtung kann anhand des gemessenen Prozessparameter entsprechende Maßnahmen einleiten. Dies kann beispielsweise eine Anpassung des Prozesses, beispielsweise ein Waschprozess sein. Ferner könnte auch ein entsprechendes Ausgabesignal für einen Nutzer ausgegeben werden. Hierzu kann die Steuerungseinrichtung mit einer Ausgabeeinrichtung und/oder einer Kommunikationseinrichtung signaltechnisch verbunden sein. Das Ausgabesignal kann beispielsweise ein visuelles oder akustisches Signal sein. Mittels der Kommunikationseinrichtung kann beispielsweise eine Nachricht an ein Nutzergerät eines Nutzers, beispielsweise ein Smartphone ein Laptop oder Ähnliches gesandt werden.

Die Anmelderin behält sich vor, sämtliche in den Anmeldungsunterlagen offenbarten Merkmale als erfindungswesentlich zu beanspruchen, sofern sie einzeln oder in Kombination gegen-über dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weitere Merkmale aus dieser Figur vorteil-haft sein kann. Ferner erkennt der Fachmann, dass sich auch Vorteile durch eine Kombination mehrerer, in einzelnen oder in unterschiedlichen Figuren gezeigten Merkmale ergeben können.

### Bezugszeichenliste

- 1: Sensorvorrichtung
- 2: Gehäuse
- 2a: oberer Abschnitt des Gehäuses
- 2b: unterer Abschnitt des Gehäuses
- 3: Leiterplatte
- 3a: fingerartige Abschnitte der Leiterplatte
- 3b: Hauptabschnitt der Leiterplatte
- 4: erste Sensoranordnung
- 5: zweite Sensoranordnung
- 6: Halterelement
- 7: stiftartiges Kontaktelement
- 8: erste Sensoreinrichtung
- 9: zweite Sensoreinrichtung
- 10: Messraum
- 11: dritte Sensoreinrichtung
- 11a: Hauptabschnitt
- 11b: Befestigungsabschnitt
- 11c: Aufnahmeabschnitt
- 11d: Übergangsabschnitt
- 11e: Endabschnitt
- 12: vierte Sensoreinrichtung
- 12a: Hauptabschnitt
- 12b: Befestigungsabschnitt
- 12c: Aufnahmeabschnitt
- 12d: Übergangsabschnitts
- 12e: Endabschnitt
- 13: zweites Kontaktelement
- 14: turmartige Elemente
- 14a: Aufnahme
- 15: Öffnung in Gehäuse
- 16: Dichtelement
- 17: Dichtelement
- 18: Öffnung
- 19: Steckerelement
- 20: Rastabschnitt des Halterelements
- 21: Durchgangskanal abschnitt des Halterelements
- 22: Befestigungselement
- 23: Abstand
- 24: Aufnahme
- 25: Durchgangskanal
- 24a: erster Abschnitt der Aufnahme
- 24b: zweiter Abschnitt der Aufnahme
- 24c: dritter Abschnitt der Aufnahme
- 25: erster Durchgangskanal in dem Halterelement
- 26: zweite Aufnahme
- 26: zweiter Durchgangskanal
- 27: Rastvorsprung
- 28: Kontaktflächen
- 29: stiftartige Elemente
- 30: erste Bohrungen der Leiterplatte
- 31: zweite Bohrungen der Leiterplatte
- 32: hakenartigen Vorsprünge 33
- 100: Haushaltsgerät
- 101: Prozessraum
- X: Längsachse
- Y: Breitenachse
- Z: Höhenachse

## Patentansprüche

1. Sensorvorrichtung (1) insbesondere für ein wasserführendes Haushaltsgerät (100) zur Detektion zumindest eines Prozessparameters, umfassend ein Gehäuse (2), in dem zumindest eine Leiterplatte (3), eine erste Sensoranordnung (4) und eine zweite Sensoranordnung (5) angeordnet sind,
**dadurch gekennzeichnet, dass**
die erste Sensoranordnung (4) auf der zumindest einen Leiterplatte (3) angeordnet ist, wobei die zweite Sensoranordnung (5) entlang einer Höhenachse (Z) von der ersten Sensoranordnung (4) beabstandet ist, wobei ein Halterelement (6) auf der zumindest einen Leiterplatte (3) angeordnet ist, an oder in welchem zumindest ein stiftartiges Kontaktelement (7) angeordnet ist, wobei das zumindest eine stiftartige Kontaktelement (7) dazu bestimmt und geeignet ist eine elektrische Verbindung zwischen der zweiten Sensoranordnung (5) und der zumindest einen Leiterplatte (3) bereitzustellen.

2. Sensorvorrichtung (1) nach Anspruch 1
**dadurch gekennzeichnet, dass**
die erste Sensoranordnung (4) zumindest eine erste Sensoreinrichtung (8) und zumindest eine zweite Sensoreinrichtung (9) umfasst, wobei die zumindest eine erste Sensoreinrichtung (8) und zumindest eine zweite Sensoreinrichtung (9) entlang einer Breitenachse (Y) beabstandet sind, wobei zwischen der zumindest einen ersten Sensoreinrichtung (8) und zumindest einen zweiten Sensoreinrichtung (9) ein Messraum (10) definiert ist.

3. Sensorvorrichtung (1) nach Anspruch 2
**dadurch gekennzeichnet, dass**
die zumindest eine erste Sensoreinrichtung (8) eine Strahlungsquelleneinrichtung ist, wobei die zumindest eine zweite Sensoreinrichtung (9) eine Detektionseinrichtung ist, wobei die Strahlungsquelleneinrichtung zumindest eine elektromagnetische Strahlung in den Messraum (10) emittiert, wobei die Detektionseinrichtung eine aus dem Messraum (10) austretende elektromagnetische Strahlung detektiert.

4. Sensorvorrichtung (1) nach einem der vorhergehenden Ansprüche,
**dadurch gekennzeichnet, dass**
die zweite Sensoranordnung (5) zumindest eine dritte Sensoreinrichtung (11) und zumindest eine vierte Sensoreinrichtung (12) umfasst, wobei die zumindest eine dritte Sensoreinrichtung (11) und zumindest eine vierte Sensoreinrichtung (12) entlang der Breitenachse (Y) beabstandet sind, wobei zwischen der zumindest einen ersten Sensoreinrichtung (11) und zumindest einen zweiten Sensoreinrichtung (12) der Messraum (10) definiert ist.

5. Sensorvorrichtung (1) nach Anspruch 4,
**dadurch gekennzeichnet, dass**
die zweite Sensoranordnung (5) dazu eingerichtet und bestimmt ist einen elektrischen Prozessparameter einer sich in dem Messraum (10) befindlichen Prozessflüssigkeit zu bestimmen, wobei die zumindest eine dritte Sensoreinrichtung (11) und die zumindest eine vierte Sensoreinrichtung (12) als Elektroden ausgebildet sind, wobei der elektrische Prozessparameter ein elektrischer Leitwert und/oder eine elektrische Kapazität ist.

6. Sensorvorrichtung (1) nach einem der Ansprüche 4 bis 5
**dadurch gekennzeichnet, dass**
die dritte Sensoreinrichtung (11)und die vierte Sensoreinrichtung (12) dazu bestimmt und eingerichtet sind die Prozessflüssigkeit in dem Messraum (10) direkt zu kontaktieren, wobei die dritte Sensoreinrichtung (11) und die vierte Sensoreinrichtung (12) im Wesentlichen stabartig ausgebildet sind.

7. Sensorvorrichtung (1) nach einem der Ansprüche 4 bis 6
**dadurch gekennzeichnet, dass**
das zumindest eine stiftartige Kontaktelement (7) an der Leiterplatte (3) angeordnet ist und sich ausgehend von der Leiterplatte (3) entlang der Höhenachse (Z) erstreckt, wobei der zumindest einen dritten Sensoreinrichtung (11) und der zumindest einen vierten Sensoreinrichtung (12) jeweils zumindest ein stiftartiges Kontaktelement (7) zugeordnet ist.

8. Sensorvorrichtung (1) nach Anspruch 7
**dadurch gekennzeichnet, dass**
jeweils zwischen einem stiftartigen Kontaktelement (7) und einer Sensoreinrichtung (11, 12) der zweiten Sensoranordnung (5) ein zweites Kontaktelement (13) angeordnet ist, wobei das zweite Kontaktelement (13) eine elektrische Verbindung zwischen dem jeweiligen stiftartigen Kontaktelement (7) und der jeweiligen Sensoreinrichtung (11, 12) bereitstellt, wobei das zweite Kontaktelement (13) als ein elastisches Element, wobei das elastische Element ein Federelement ist.

9. Sensorvorrichtung (1) nach einem der Ansprüche 2 bis 8,
**dadurch gekennzeichnet, dass**
das Gehäuse (2) einen oberen Abschnitt (2a) aufweist, in welchem turmartige Elemente (14) angeordnet sind, wobei die turmartigen Elemente (14) entlang der Breitenachse (Y) sich gegenüberliegend angeordnet sind, wobei die zumindest eine erste Sensoreinrichtung (8) und zumindest eine zweite Sensoreinrichtung (9) jeweils in einem der turmartigen Elemente (14) angeordnet sind, wobei die Leiterplatte (3) zwei fingerartige Abschnitte (3a) aufweist, welche sich entlang einer Längsrichtung (X) erstrecken, wobei jeweils ein fingerartiger Abschnitt (3a) in einem turmartigen Element (14) angeordnet ist.

10. Sensorvorrichtung (1) nach Anspruch 9,
**dadurch gekennzeichnet, dass**
die turmartigen Elemente (14) jeweils eine Aufnahme (24) aufweisen, wobei die dritte Sensoreinrichtung und die vierte Sensoreinrichtung jeweils in einer Aufnahme (14a) angeordnet sind, wobei die dritte Sensoreinrichtung (11) und die vierte Sensoreinrichtung (12) jeweils durch eine Öffnung (15) in dem oberen Abschnitt (2a) des Gehäuses (2) sich erstrecken, wobei die dritte Sensoreinrichtung (11) und die vierte Sensoreinrichtung (12) jeweils ein Dichtelement (16) aufweisen, welches zwischen der jeweiligen Sensoreinrichtung (11, 12) und der jeweiligen Öffnung (15) angeordnet ist.

11. Sensorvorrichtung (1) nach einem der Ansprüche 9 bis 10
**dadurch gekennzeichnet, dass**
ein unterer Abschnitt (2b) des Gehäuses (2) an den oberen Abschnitt (2a) des Gehäuses (2) anschließt, wobei in einem oberen Bereich des unteren Abschnitts (2b) des Gehäuses (2) ein Dichtelement (17) an einer äußeren Wandung des unteren Abschnitts (2b) angeordnet ist, wobei der untere Abschnitt (2b) eine Öffnung (18) aufweist, durch welche ein Steckerelement (19) in das Gehäuse (2) einführbar ist.

12. Sensorvorrichtung (1) nach einem der vorhergehenden Ansprüche,
**dadurch gekennzeichnet, dass**
das Halterelement (6) einen Rastabschnitt (20) aufweist, welcher ein einem an der Leiterplatte (3) anordenbaren Steckerelement (19) einrastbar ist.

13. Sensorvorrichtung (1) nach einem der vorhergehenden Ansprüche,
**dadurch gekennzeichnet, dass**
das Halterelement (6) zumindest einen Armabschnitt (21) aufweist, wobei der zumindest eine Armabschnitt (21) ein Befestigungselement (22) aufweist, welches den Armabschnitt (21) an der Leiterplatte (3) befestigt.

14. Haushaltsgerät (1) umfassend zumindest eine Sensorvorrichtung nach einem der vorhergehenden Ansprüche.
